Europäisches Patentamt

(19) European Patent Office

Office européen des brevets

(11) Publication number: **0 243 717**
**A1**

(12) **EUROPEAN PATENT APPLICATION**

(21) Application number: **87104906.0**

(22) Date of filing: **02.04.87**

(51) Int. Cl.³: **C 07 H 15/203**
**A 61 K 31/70, A 61 K 31/05**

(30) Priority: **03.04.86 JP 75617/86**

(43) Date of publication of application:
**04.11.87 Bulletin 87/45**

(84) Designated Contracting States:
**AT BE CH DE ES FR GB GR IT LI LU NL SE**

(71) Applicant: **THE KITASATO INSTITUTE**
**9-1 Shirogane 5-chome Minato-ku**
**Tokyo(JP)**

(72) Inventor: **Omura, Satoshi**
**5-12-7, Seta Setagaya-ku**
**Tokyo(JP)**

(72) Inventor: **Nakagawa, Akira**
**2-30-31, Sumiyoshi-cho**
**Fuchu-shi Tokyo(JP)**

(74) Representative: **Wächtershäuser, Günter, Dr.**
**Tal 29**
**D-8000 München 2(DE)**

(54) Use of hygromycin a in the therapy and prophylaxis of swine dysentery.

(57) This invention relates to methods of using hygromycin A for the treatment and prevention of swine dysentery, and the therapeutic compositions which contain hygromycin A as the effective ingredient.

EP 0 243 717 A1

Croydon Printing Company Ltd.

## USE OF HYGROMYCIN A IN THE
## THERAPY AND PROPHYLAXIS OF SWINE DYSENTERY

### BACKGROUND OF THE INVENTION

Swine dysentery is an acute or chronic intestinal infestation accompanied by mucosanguineous diarrhea, a disease infesting swine during their growth. The principal causative agent is Treponema hyodysenteriae with other enteric involved in the microorganisms outbreak of the disease. Because recently introduced mass husbandry practices employed for swine have increased the incidence of the disease, the resulting morbidity and mortality are steadily increasing.

It is known that the infection is transmitted orally, with the feces of infected or sick swine serving as the source of infection. Since the feces contains a large number of pathogens, the environment is rapidly contaminated, exposing all the swine raised in the same facility.

Swine dysentery, has been treated by the administration of naturally-occurring antibiotics or synthetic antibacterials. For example, such antibiotics as tiamulin (DYNAMUTILIN®) lankacidin, LINCOMYCIN®), and the synthetic antibacterial, carbadox (which however, has toxic side effects) have been shown to have some effect on Treponema hyodysenteriae.

Penicillin, aminoglycosides, tetracycline, chloramphenicol, macrolides and the like, have also been effective in treating swine for other bacterial diseases, including diseases causing diarrhea as have antibacterial drugs, such as olaquindox, sulfa drugs and nitrofans.

Aforenamed drugs are acknowledged to be effective against bacterial diarrhea of swine to a certain degree, but also present a number of problems. The antibacterials effective against Treponema hyodysenteriae, the principal pathogen of swine dysentery, are few and of varying efficacy. Moreover, frequent use of those drugs raises the probability of the appearance of resistant bacteria. Thus development of a new drug is now keenly demanded, especially one combining mineral residual toxicity with superior efficacy.

After screening metabolites several thousand strains of Actinomycetes for therapeutic and prophylactic activity against swine dysentery, we discovered that the antibiotic hygromycin A, produced in the culture medium of Actinomyces, Streptomyces hygroscopicus, is extremely effective both as a therapeutic and prophylactic drug against Treponema hyodysenteriae, the principal infectious agent in swine dysentery.

### BRIEF DESCRIPTION OF THE INVENTION

A primary object of the present invention is to provide curative and preventive compositions for swine dysentery, which contain as their effective ingredient hydromycin A.

Another object of the present invention is to provide both therapeutic and prophylactic treatment methods for swine dysentery, which comprise administering compositions containing therapeutic or prophylactic amounts of hygromycin A to swine.

## DETAILED DESCRIPTION OF THE INVENTION

Hygromycin A has been known as an antibiotic produced by Streptomyces hygroscopicus, which has been referred to as effective against some Gram-positive bacteria mycoplasma. its preparation method and properties are shown in Antibiotics & Chemotherapy, 3, 1268 (1953).

Hygromycin A is an amino-cyclitol antibiotic containing a glycoside and an aromatic ring. Therefore it is suitably administered as a curative or preventive composition against swine dysentery, either as a liquid formulation dissolved in drinking water, or as an additive to be mixed into the swine feed.

Most preferably, therapeutic administration to swine is in the form of an aqueous solution of hygromycin A, both because the swine require water to overcome their dysentery-induced dehydration and also because of convenience and efficiency of this method in swine stock management. It could theoretically be used as an injectable, but because swine dysentery generally takes the form of mass outbreak, and, in any event, even if only a minor number of swine are infected, injections are labor-intensive and expensive method and the drug must be administered as a preventive drug during the latent period to all exposed swine.

Hygromycin A may be dissolved in drinking water of swine alone, or with the optional addition of other drugs for stabilization, such as a buffer, antioxidants and the like. Flavoring agents may also be added. If the drug taste prove aversive, needless to say, hygromycin A concentration in the drinking water should be varied according to the physical conditions of the swine, i.e., their water-drinking rate, to achieve an effective hygromycin A concentration in their digestive organ.

When used as a feed additive, hygromycin A powder may be mixed into the feed either as it, or with an excipient when it is mixed immediately before feeding. When it is mixed in the feed in advance, its hygroscopicity may be taken into consideration. Also a stabilizer or the like may be added to prevent its decomposition when the feed is kept warm. Microencapsulated formulation may also be effective.

To achieve a suitable dosage range, i.e., 0.02-3.0 mg/kg, the drug may be dissolved in the swine drinking water at a concentration of 0.2-30 mg/liter. Should it be administered as an intramuscular injection, a 50 mg/ml aqueous solution or physiological saline solution of hygromycin A can be injected to effect administration of the 0.02-3 mg/kg of swine weight dosage.

For administration as a food additive, the hygromycin A concentration in the feed can be determined similarly to the case of administration through drinking water. Normally it can be 0.2-20 mg/kg feed, preferably 1-10 mg/kg feed. Gavage or injection are recommended for suckling pigs. With young pigs, outbreak of dysentery necessitates administration of a therapeutically effective dosage to all the pigs being raised in the same quarters, and therefore use of hygromycin A as a feed additive or drinking water additive is recommended. Because hygromycin A has extremely low toxicity compared with heretofore known therapeutic or prophylactic drugs for swine dysentery, the problem of residual toxicity is negligible.

Hygromycin A also found to be effective against Haemophilus pleuropneumoniae KB282, a pathogen of pleuropneumonia which is a serious swine disease and Pasteurella haemolytica, one of the pathogens of pasteurellosis known as swine plague in our screening systems.

Hereinafter the results of therapeutic experiments of laboratory infected animals, using hygromycin A will be shown.

Groups of seven-week old mice (CF-1) are orally infected with Treponema hyodensenteriae DJ 70 P-3 as the test pathogen, each group consisting of five mice. The mice are fasted beginning one day before the bacterial infection, and hygromycin A is administered to them in drinking water at the dosages of 60, 30, 15, 7.5, 3.7, 1.8 and 0.9 $\mu$g/ml. The bacterial infection is effected on the first and second days after fasting. The drug is administered through drinking water for six consecutive days after the first bacterial infection. On the sixth day of the administration the test animals are necropsied, and presence of Treponema hyodysenteriae in the contents of their digestive organs are determined by three days' cultivation in blood agar. As the positive control, Lincomycin (60-3.7 $\mu$g/ml) Dynamutilin, lankacidin C and olaquindox (60-7.5 $\mu$g/ml, respectively) are administered through drinking water at the concentrations indicated. As the negative control, untreated group allowed to freely drink water. The results are shown in Table I.

Table 1

| Drug | Dosage ($\mu g/m\ell$) | Lesion | Presence of T. hyo. | | Noninfective (%) | ED$_{50}$ ($\mu g/m\ell$) |
|---|---|---|---|---|---|---|
| | | | 1st day | 3rd day | | |
| Hygromycin A | 60 | 0/5 | 0/5 | 0/5 | 100 | |
| | 30 | 0/5 | 0/5 | 0/5 | 100 | |
| | 15 | 0/5 | 0/5 | 0/5 | 100 | 1. 1 |
| | 7. 5 | 0/5 | 0/5 | 0/5 | 100 | |
| | 3. 7 | 0/5 | 0/5 | 0/5 | 100 | |
| | 1. 8 | 0/5 | 0/5 | 0/5 | 100 | |
| | 0. 9 | 0/5 | 0/5 | 3/5 | 40 | |
| Lincomycin | 60 | 0/3 | 0/3 | 0/3 | 100 | |
| | 30 | 0/5 | 0/5 | 0/5 | 100 | |
| | 15 | 0/5 | 0/5 | 2/5 | 60 | 13 |
| | 7. 5 | 0/5 | 0/5 | 4/5 | 20 | |
| | 3. 7 | 1/5 | 3/5 | 5/5 | 0 | |
| Dynamutilin | 60 | 0/5 | 0/5 | 0/5 | 100 | |
| | 30 | 0/5 | 0/5 | 3/5 | 40 | 40 |
| | 15 | 0/5 | 0/5 | 4/5 | 20 | |
| | 7. 5 | 3/5 | 5/5 | 5/5 | 0 | |

Table 1 ( continued )

| | | | | | | |
|---|---|---|---|---|---|---|
| Lankacidin C | 60 | 0／5 | 0／5 | 1／5 | 80 | |
| | 30 | 0／5 | 0／5 | 2／5 | 60 | |
| | 15 | 0／5 | 0／5 | 5／5 | 0 | 40. 5 |
| | 7. 5 | 3／5 | 3／5 | 5／5 | 0 | |
| Olaquindox | 60 | 3／5 | 3／5 | 5／5 | 0 | |
| | 30 | 5／5 | 5／5 | 5／5 | 0 | |
| | 15 | 5／5 | 5／5 | 5／5 | 0 | ＞60 |
| | 7. 5 | 5／5 | 5／5 | 5／5 | 0 | |
| Control | 0 | 5／5 | 5／5 | 5／5 | 0 | |

Method

Mouse C F－1   Seven weeks, female ( 5 mice per group )

Test pathogen : Treponema   hyodysenteriae  D J 7 0  P－3

Drugs          : Hygromycin  A   6 0、3 0、1 5、7. 5、3. 7、1. 8、0. 9 $\mu$ g／m $\ell$

Lincomycin     6 0、3 0、1 5、7. 5、3. 7 $\mu$ g／m $\ell$

Dynamutilin    6 0、3 0、1 5、7. 5、3. 7 $\mu$ g／m $\ell$

Lankacidin  C  6 0、3 0、1 5、7. 5 $\mu$ g／m $\ell$

Olaquindox     6 0、3 0、1 5、7. 5 $\mu$ g／m $\ell$

024371 7

Table 1 ( continued )

```
                    -1    0    1    2    3    4    5    6    7    8    9 ( day )


Administration
through
drinking water
        Fasting
        T.  hyo.                              Dissection
                                                        Evaluation   Evaluation
                                                        by culture   by culture
```

Curative ratios of the three drugs calculated
from the foregoing results are as in Table 2.

Table 2

| Dosage | Hygromycin A | Lincomycin | Lankacidin C | Olaquindox | Dynamutilin |
|--------|--------------|------------|--------------|------------|-------------|
| μg／mℓ | % | % | % | % | % |
| 6 0 | 1 0 0 | 1 0 0 | 8 0 | 0 | 1 0 0 |
| 3 0 | 1 0 0 | 1 0 0 | 6 0 | 0 | 4 0 |
| 1 5 | 1 0 0 | 6 0 | 0 | 0 | 2 0 |
| 7. 5 | 1 0 0 | 2 0 | 0 | 0 | 0 |
| 3. 7 | 1 0 0 | 0 | | | |
| 1. 8 | 1 0 0 | | | | |
| 0. 9 | 4 0 | | | | |

0243717

Fifty-percent effective dosages ($ED_{50}$) with CF-1 mice of the five drugs by administration through drinking water calculated from the foregoing results are as follows.

| | | |
|---|---|---|
| Hygromycin A | 1.1 | μg/ml |
| Lincomycin | 13 | μg/ml |
| Dynamutilin | 40 | μg/ml |
| Lankacidin C | 40.5 | μg/ml |
| Olaquindox | >60 | μg/ml |

For reference, the minimum growth inhibition concentrations (MIC) to various bacteria of above five drugs and Lankacidin C are shown in Table 3.

T a b l e  3

| Test bacteria | Medium[a] | MIC (μg/ml) | | | |
|---|---|---|---|---|---|
| | | Hygromycin A | Lincomycin | Dynamutilin | Lankacidin C |
| _Staphylococcus_ _aureus_ KB 210 (ATCC 6538p) | A | 50 | 1.56 | 0.78 | 0.4 |
| _S._ _aureus_ KB 34 (FDA 209p) | A | 50 | 3.12 | 3.12 | 1.56 |
| _S._ _aureus_ KB 199 (ML [r],TC [r]) [b] | A | 50 | >100 | 3.12 | 1.56 |
| _S._ _aureus_ KB 283 (MRSA) [s] | A | 50 | 6.25 | 3.12 | 3.12 |
| _S._ _aureus_ KB 285 (MRSA) [c] | A | 50 | 12.5 | 3.12 | 3.12 |
| _Bacillus_ _subtilis_ KB 211 (ATCC 6633) | A | >100 | 50 | >100 | >100 |
| _B._ _cereus_ KB 143 (IFO 3001 | A | >100 | 25 | >100 | 100 |
| _B._ _megaterium_ KB 144 (IFO 12108) | A | 100 | >100 | 100 | >100 |
| _Micrococcus_ _luteus_ KB 212 (ATCC 9341) | A | 25 | 0.4 | 0.78 | < 0.2 |
| _Mycobacterium_ _smegmatis_ KB 42 (ATCC 607) | A | >100 | >100 | >100 | >100 |
| _Escherichia_ _coli_ KB 213 (NIHJ) | A | 100 | >100 | >100 | >100 |
| _E._ _coli_ KB 176 (NIHJ JC-2) | A | >100 | >100 | >100 | >100 |
| _E._ _coli_ KB 198 (ML [r],LCM[s]) [d] | A | 12.5 | 3.12 | 6.25 | 0.78 |
| _Klebsiella_ _pneumoniae_ KB 214 (IFO 10031) | A | 50 | >100 | 100 | >100 |
| _Proteus_ _vulgaris_ KB 127 (IFO 3167) | A | 50 | >100 | >100 | >100 |
| _Pseudomonas_ _aeruginosa_ KB 115 (IFO 3080) | A | >100 | >100 | >100 | >100 |
| _Xanthomonas_ _oryzae_ KB 88 | E | 50 | >100 | < 3.12 | < 3.12 |
| _Haemophilus_ _pleuropneumoniae_ KB 282 | C | 12.5 | 50 | 50 | 6.25 |
| _pasteurella_ _hemolytica_ KB 281 | C | 25 | >100 | >50 | 25 |

0243717

Table 3 ( continued )

| | | | | | |
|---|---|---|---|---|---|
| Clostridium perfringens KB 129 (ATCC 3624) | B | 6.25 | 0.4 | 0.78 | 3.12 |
| C. kainantoi KB 133 (IFO 3353) | B | 3.12 | 3.12 | 1.56 | 3.12 |
| C. novyi KB 136 (ILD 140) | B | 3.12 | < 0.2 | < 0.2 | 0.4 |
| C. difficile KB 258 (ATCC 9689) | B | 25 | 6.25 | 12.5 | 25 |
| Bcteroides fragilis KB 169 (ATCC 23745) | B | 50 | 0.4 | < 0.2 | 0.78 |
| Fusobacterium varium KB 234 (ATCC 8501) | B | 25 | 12.5 | >100 | >100 |
| Peptococcus variabilis KB 235 (ATCC 14955) | B | 3.12 | 0.78 | 0.4 | 0.4 |
| Mycoplasma gallisepticum KB 170 (KP-13) | D | 0.78 | 12.5 | < 0.05 | 50 |
| M. gallisepticum KB 171 (S-6) | D | 0.78 | 12.5 | < 0.05 | 25 |
| M. gallisepticum KB 172 (333-P) | D | 1.56 | >50 | < 0.05 | 50 |
| M. pneumoniae KB-173 | D | 0.78 | 25 | < 0.05 | 25 |
| Acholeplasma laidlawii KB 174 (PG-8) | D | 0.78 | 12.5 | < 0.05 | 25 |
| A. laidlawii KB 175 (Bm-1) | D | 0.78 | 12.5 | < 0.05 | 25 |
| Candida albicans KF-1 | E | >100 | >100 | >100 | >100 |
| Saccharomyces sake KF 26 | E | >100 | >100 | >100 | >100 |
| S. cerevisiae KF 237 (ATCC 9763) | E | >100 | >100 | >100 | >100 |
| Aspergillus niger KF-105 | E | >100 | >100 | >100 | >100 |
| piricularia oryzae KF 180 | E | >100 | >100 | >100 | >100 |
| Trichophyton interdigitale KF 62 | E | >100 | >100 | >100 | >100 |
| Mucor racemosus KF 223 (IFO 4581) | E | >100 | >100 | >100 | >100 |
| Penicillium herquei KE 227 (IFO 4647) | E | >100 | >100 | >100 | >100 |
| Treponema hyodysenteriae B 78 | F | 6.25 | 1.56 | 0.20 | 0.78 |
| Treponema hyodysenteriae DJ 70 P-3 | F | 3.12 | 0.20 | 0.20 | 0.01 |

a

A:   Sensitivity test agar medium (Nissui), $37^{O}C$, 20 hrs.

B:   GAM agar medium, anaerobic condition, $37^{O}C$, 24 hrs.

C:   S-medium, $Co_2$ (5%), $37^{O}C$, 20 hrs.

D:   PPLO agar medium (Difco), $35^{O}C$, 6 hrs.

E:   Potato-glucose agar medium, $27^{O}C$, 72 hrs.

F:   Tripticase-soy agar medium containing 5% sheep blood

b

$ML\gamma$: Macrolide resistance

$TC\gamma$: Tetracycline resistance

c

MRSA:   Methicillin resistant <u>Staphylococcus aureus</u> ($\beta$-lactamase-positive methicillin resistance)

d

$ML\gamma$: Macrolide resistance

$LCM^{S}$ : Lincomycin susceptibility

Hereinafter the present invention will be explained with reference to the examples, it being understood that the invention is not to be construed as limited thereby.

**Example 1**

Additive to drinking water:

One (1) mg of hygromycin A is dissolved in 1 liter of sterilized distilled water to form a drug to be administered through drinking water. Sugar is added optionally to sweeten the formulation.

**Example 2**

Feed additive:

Five (5) g of hygromycin A are mixed thoroughly with 900 g of corn starch as an excipient and 95 g of calcium carbonate in a stirrer to form a hygromycin A feed additive (premix). This additive is to be mixed with swine feed at a ratio of 200 g/ton immediately before the feeding.

**Example 3**

Injection:

Fifty (50) g of hygromycin A are dissolved in 1 liter of sterilized physiological saline solution, passed through a millipore filter and aseptically poured into 20 ml ampoules to produce hygromycin A injection dosages, to be intramuscularly injected to an animal suffering from swine dysentery at a dosage of 0.5 mg/kg.

Based on the data set forth above, we hypothesized that if we should run the experiments of Examples 4 and 5, we would obtain the results set out in Table 4-Table 8.

Example 4

Therapeutic experiment of swine dysentery [illustrated by modified Am. J. Vet. Res., 41 (9), 1349 (1980)]:

Pathogenic Treponema hyodysenteriae isolated from feces of animals suffering from swine dysentery and sub-inoculated to mice 15 times are cultured.

Thirty-six, 5 to 6 week-old male pigs, raised for 21 days under quarantine, are used in the following experiment, after it is confirmed with rectal swabs that no Treponema hyodysenteriae and Salmonella sp. are detected.

The test animals are divided into three twelve-membered groups, and put into separate rooms for each group. The excrement is cleaned every day during the raising. The animals are fasted for 48 hours before the bacterial infection.

Two groups are orally infected with Treponema hyodysenteriae culture liquid in an amount of 100 ml per pig. The remaining group is the control, and receive the same amount of sterilized culture medium. All three groups are allowed to freely eat the swine feed. The pharmacotherapeutic method is as indicated in Table 4 below.

<u>T a b l e  4</u>

| Group | Treatment |
|---|---|
| I | Raised for 125 days, not infected, no treatment given |
| I I | Raised for 125 days, infected, no treatment given |
| I I I | Raised for 125 days, infected, hygromycin A ( added to drinking water at a ratio of 0.5m g／liter, free intake ) was administered for 30 consecutive days starting on the third day after the infection.  No subsequent treatment given |

The general symptoms and feces condition of the test animals are observed and recorded daily. The infectious pathogens are collected with rectal swabs once a week, to check for the presence of Treponema hyodysenteriae and Salmonella sp.

After the experiment, the test animals are necropsied. Thereafter, their colons are collected for pathological examination. The results are shown in Tables 5 and 6.

<u>T a b l e  5  Clinical Test Results</u>

| Group | Number of pigs | Average diarrhea index* | | Change in morbidity (%) | | Mortality |
| --- | --- | --- | --- | --- | --- | --- |
| | | Pre-medication | During medication | Third day | Thirtieth day | |
| I | 1 2 | 0 | 0. 1 1 | 0 | 0 | 0 |
| I I | 1 2 | 0 | 1. 4 | 7／1 2 | 0／7 | 5／1 2 |
| I I I | 1 2 | 0 | 0. 2 2 | 0 | 0 | 0 |

\* 0 : normal , 1 : diarrhea , 2 : feces cruentae

<u>T a b l e  6  Pathogen Detection Rate ( % )</u>

| Group | Prein-fecting | Post infecting days 1 to 3 Before medication | During medication days 31 to 120 | Withdrawal days 31 to 120 |
| --- | --- | --- | --- | --- |
| I | 0／1 2 | 0／1 2 | 0／1 2 | 0／1 2 |
| I I | 0 | 7／1 2 | 1 0／1 2 | 0／7 |
| I I I | 0 | 1 0／1 2 | 0／1 2 | 0／1 2 |

Gross pathological examination of the colon reveals that the control (non-infected, non-treated) groups and the infected, therapeutically treated groups are normal, but the infected, non-treated group shows congestion and edema of the intestinal walls, the typical swine inflammation symptom.

Example 5

Prevention of swine dysentery infection:

Fifteen female and male pigs in total (5-6 weeks age) are divided into three five-membered groups, and each group housed in a separate room. Group I is non-infected and non-treated group; Group II, infected and non-treated group; and Group III, infected and therapeutically treated group (hygromycin A 0.5 mg/liter of drinking water, free intake). Groups II and III are orally infected with 100 ml of Treponema hyodysenteriae culture liquid per pig on the third day after drug administration started. Group III only continuously receives the drug for the following 20 consecutive days. Thereafter the pigs of all three groups are washed, sterilized and transferred into each an asceptic pen. Five healthy pigs of same kind, confirmed to be uninfected, are then placed in the same pen, making each group ten-membered. All groups are raised for the following 93 days, during which the change in morbidity and mortality are observed starting from the date on which the healthy pigs were introduced (after 29 days of therapy). The method of observation is similar to that employed in Example 4.

No attack of the disease is observed with Group I. In contrast thereto, in Group II, four out of five pigs infected at the beginning of the start of the observation period recovered. The remaining one still shows the symptoms of swine dysentery. During the observation period, the last pig recovers, but two out of the other four relapse and three out of five healthy pigs have an attack of the disease. In Group III, infected pigs show no swine dysentery symptoms during the therapeutic period and at the starting time of observation. During the observation period no attack of the disease is observed with any of the infected pigs or healthy pigs. (Table 7 and 8). The foregoing result demonstrates that hygromycin A is effective for prevention of swine dysentery or passing the infection thereof to other healthy pigs.

Table 7 Clinical Test Results

| Group | Number of Pigs | Average diarrhea index * | | | Change in morbidity ( % ) | | | Mortality (%) |
|---|---|---|---|---|---|---|---|---|
| | | pre-medication | During medication | During observation | During medication | Commencement of observation | During observation | |
| I | 5 | 0 | 0 | 0 | 0 | 0 | 0 | 0 |
| | 5 ** | | | 0 | | 0 | 0 | 0 |
| II | 5 | 0 | 1. 7 | 1. 2 | 5／5 | 1／5 | 3/5(～2/5) | 0 |
| | 5 ** | | | 1. 5 | | 0／5 | 3／5 | 0 |
| III | 5 | 0 | 0 | 0 | 0／5 | 0 | 0 | 0 |
| | 5 ** | | | 0 | | 0 | 0 | 0 |

0 : normal , 1 : diarrhea , 2 : feces cruentae : ** : additional healty pigs

Table 8 Pathogen Detection Rate ( % )

| group | Number of Pigs | pre-infection | During medication | Commencement of observation | During observation |
|---|---|---|---|---|---|
| I | 5 | 0 | 0 | 0 | 0 |
| | 5 | | | 0 | 0 |
| II | 5 | 0 | 5／5 | 4／5 | 5／5 |
| | 5 | | | 0 | 5／5 |
| III | 5 | 0 | 0 | 0 | 0 |
| | 5 | | | 0 | 0 |

The studies to determine the efficacy of hygromycin A for the control of swine dysentery using a controlled, experimentally-induced clinical disease trial in swine, in fact, are set out in Example 6 and Example 7.

**Example 6**

Therapeutic experiment of swine dysentery with feed additives:

Fifty crossbred barrows and gilts, 6 weeks of age, average body weight of 10.7 kg, 5 pigs per group, are divided into five groups (A, B, C, D and E), each consisting of 2 groups, total 10 pigs in each group. Hygromycin A is administered as follows: in group A, hygromycin A 10 g/ton feed; group B, hygromycin A 5 g/ton feed; group C, hygromycin A 1 g/ton feed; group D, carbadox 50 g/ton feed; and group E, control, feed containing no drug. (Table 9).

T a b l e  9    Experimental Design

| Treatment Designation | Drug in Feed | Drug Level ( g／T) | Induced Dysentery | Number of Group | Number of pigs per group | Total Number of pigs Treatment |
|---|---|---|---|---|---|---|
| A | Hygromycin  A | 1 0 | Infected | 2 | 5 | 1 0 |
| B | Hygromycin  A | 5 | Infected | 2 | 5 | 1 0 |
| C | Hygromycin  A | 1 | Infected | 2 | 5 | 1 0 |
| D | Carbadox | 5 0 | Infected | 2 | 5 | 1 0 |
| E | Control | 0 | Infected | 2 | 5 | 1 0 |

All pigs are raised under normal conditions for 4 days, and after it is confirmed that no _Treponema hyodysenteria_ and _Salmonella sp._ can be detected, the animals are fasted for 6 hours without drinking water. All pigs are orally infected with _Treponema hyodysenteriae_ B 204 culture liquid in an amount of 100 ml per pig. Respective treatment diets are provided 1 hour postchallenge and continued _ad libitum_ for 2 weeks followed by a two week nonmedication period. All pigs are necropsied on day 28 postchallenge to assess gross lesions of the large intestine. Notes and records of the clinical signs of growth condition, condition of feces and body weight are made concurrently for each pig on each day postchallenge. Feed consumption and individual pig body weight measurements and rectal swabs for _T. hyo_ isolation are taken prechallenge and then weekly postchallenge.

Results are shown in Tables 10-14.

Ta b l e  1 0  General Appearance

| Treatment Designation [a] | Group No. | General appearance : No of pig days [b] | | |
|---|---|---|---|---|
| | | Normal | Depressed | Moribund |
| A | 6 | 1 4 5 | 0 | 0 |
| | 9 | 1 4 5 | 0 | 0 |
| | Total | 2 9 0 (1 0 0 %) | 0 | 0 |
| B | 1 | 1 4 5 | 0 | 0 |
| | 8 | 1 4 5 | 0 | 0 |
| | Total | 2 9 0 (1 0 0 %) | 0 | 0 |
| C | 2 | 1 4 5 | 0 | 0 |
| | 5 | 1 4 5 | 0 | 0 |
| | Total | 2 9 0 (1 0 0 %) | 0 | 0 |
| D | 4 | 1 4 4 | 1 | 0 |
| | 7 | 1 2 8 | 2 | 0 |
| | Total | 2 7 2 (9 9 %) | 3 (1 %) | 0 |
| E | 3 | 7 9 | 3 2 | 9 |
| | 1 0 | 6 2 | 6 5 | 1 |
| | Total | 1 4 1 (5 7 %) | 9 7 (3 9 %) | 1 0 (4 %) |

Table  10  ( continued )

a)

A  =  1 0 g／T  Hygromycin  A

B  =  5 g／T  Hygromycin  A

C  =  1 g／T  Hygromycin  A

D  =  5 0 g／T  Carbadox

E  =  Nonmedicated

b)

A total of 145 pig days for each group, except : treatment E. No. 7 = 130 pig days.

Treatment E, No. 3 = 120 pig days , Treatment E, No. 10 = 128 pig days

Note : Number of pig revealed appearance × Number of days thereof

In group E, (nonmedicated group), many depressed pigs are observed and in group D, a small number of depressed pigs are seen. In groups A-C, however, most pigs were found with normal condition. Table 11 shows a fecal score. In nonmedicated group E, 65% of pigs showed not normal feces, 36% of which revealed feces cruentae with typical pig dysentery. On the contrary, a little number of pigs in groups A-D revealed soft feces, but almost thereof was normal.

Table 12 shows average pig body weight at each measurement period. All pigs showed almost same body weight before challenging _Treponema hyodysenterial_, however, at postchallenging pig groups A-C showed significantly different body weight changes as compared with pig group D, in which a decrease of feed effectiveness was prevented by administering hygromycin A as same degree as in carbadox administered group.

T a b l e　1 1　Number of pig days ( fecal score ) 1、2 、3 、4 and 5 [a]

| Treatment[b] | Group No. | No. pig days with fecal score of : [c] | | | | |
|---|---|---|---|---|---|---|
| | | 1 | 2 | 3 | 4 | 5 |
| A | 6 | 1 4 1 | 4 | 0 | 0 | 0 |
| | 9 | 1 4 2 | 3 | 0 | 0 | 0 |
| | Total | 2 8 3 ( 9 8 %) | 7 ( 2 %) | 0 | 0 | 0 |
| B | 1 | 1 4 0 | 5 | 0 | 0 | 0 |
| | 8 | 1 3 0 | 1 5 | 0 | 0 | 0 |
| | Total | 2 7 0 ( 9 3 %) | 2 0 ( 7 %) | · 0 | 0 | 0 |
| C | 2 | 1 2 9 | 1 6 | 0 | 0 | 0 |
| | 5 | 1 3 2 | 1 0 | 1 | 0 | 2 |
| | Total | 2 6 1 ( 9 0 %) | 2 6 (9 %) | 1　(0.3 %) | 0 | 2　(0.7%) |
| D | 4 | 1 3 0 | 1 5 | 0 | 0 | 0 |
| | 7 | 1 1 5 | 1 2 | 0 | 1 | 2 |
| | Total | 2 4 5 ( 8 5 %) | 2 7 ( 1 0 %) | 0 | 1　(0.3%) | 2 (0.7%) · |
| E | 3 | 4 5 | 2 2 | 7 | 1 0 | 3 6 |
| | 1 0 | 4 2 | 8 | 6 | 2 0 | 5 2 |
| | Total | 8 7　( 3 5 %) | 3 0　( 1 2 %) | 1 3　( 5 %) | 3 0　( 1 2 %) | 8 8 (36%) |

Table 11 ( continued )

a)

1 = Normal

2 = Semi-solid, pasty

3 = Watery with some solid material

4 = Profuse, Watery feces with little solid material

5 = Blood present ( any consistency )

b)

A = 1 0 g／T  Hygromycin  A

B = 5 g／T  Hygromycin  A

C = 1 g／T  Hygromycin  A

D = 5 0 g／T  Carbadox

E = Nonmedicated

c)

A total of 145 pig days for each group, except : Treatment D, No. 7 = 130 pig days;

Treatment E, group 3 = 120 pig days ; Treatment E, group 10 = 128 pig days

| Treatment[a] | Group No. | | Body weight ( kg ) at each Period | | | | | |
|---|---|---|---|---|---|---|---|---|
| | | | Adjustment Day | | Postchallenge Day | | | |
| | | | 0 | 4 | 7 | 14 | 21 | 28 |
| A | 6 | | 11. 0 | 12. 6 | 16. 0 | 20. 3 | 24. 9 | 29. 7 |
| | 9 | | 10. 3 | 11. 8 | 14. 4 | 18. 8 | 23. 1 | 28. 5 |
| | | Mean | 10. 7 | 12. 2 | 15. 2 | 19. 6 | 24. 0 | 29. 1 |
| | | ( S.D.) | (1. 16) | (1. 22) | (1. 97) | (2. 50) | (2. 97) | (3. 07) |
| B | 1 | | 10. 1 | 11. 3 | 14. 6 | 18. 4 | 22. 4 | 27. 1 |
| | 8 | | 11. 2 | 12. 6 | 15. 6 | 20. 1 | 24. 7 | 29. 1 |
| | | Mean | 10. 6 | 12. 0 | 15. 1 | 19. 2 | 23. 5 | 28. 1 |
| | | ( S.D.) | (0. 97) | (1. 05) | (1. 34) | (1. 52) | (1. 95) | (2. 04) |
| C | 2 | | 10. 3 | 11. 7 | 14. 6 | 18. 4 | 23. 1 | 27. 7 |
| | 5 | | 11. 0 | 11. 8 | 14. 9 | 19. 2 | 23. 6 | 28. 1 |
| | | Mean | 10. 7 | 11. 7 | 14. 8 | 18. 8 | 23. 3 | 27. 9 |
| | | ( S.D.) | (0. 97) | (1. 08) | (1. 26) | (1. 42) | (1. 67) | (2. 10) |
| D | 4 | | 11. 0 | 12. 2 | 15. 4 | 19. 3 | 23. 9 | 28. 9 |
| | 7 | | 10. 5 | 11. 5 | 15. 2 | 18. 2 | 24. 0[b] | 29. 7[b] |
| | | Mean | 10. 7 | 11. 9 | 15. 3 | 18. 8 | 24. 0 | 29. 3 |
| | | ( S.D.) | (1. 03) | (1. 27) | (1. 64) | (3. 10) | (2. 43) | (2. 74) |

Table 12 (continued)

| | | | | | | | |
|---|---|---|---|---|---|---|---|
| | 3 | | 11. 0 | 12. 5 | 14. 7 | 14. 6[b] | 16. 1[b] | 21. 8[c] |
| E | 10 | | 10. 3 | 11. 4 | 14. 3 | 13. 7[b] | 12. 5[b] | 9. 7[b] |
| | | Mean | 10. 7 | 11. 9 | 14. 5 | 14. 2 | 14. 3 | 14. 9 |
| | | ( S.D.) | (0. 8 8) | (1. 0 5) | (2. 2 3) | (4. 1 9) | (6. 1 0) | (8. 9 4) |

a)

A = 1 0 g／T Hygromycin A

B = 5 g／T Hygromycin A

C = 1 g／T Hygromycin A

D = 5 0 g／T Carbadox

E = Nonmedicated

Pigs were challenged with T. hyo. on day 4 and then provided respective treatment diets immediately

following challenge for a 14-day medication period followed by a 14-day nonmedication period.

b)

Mean of 4 pigs due to mortality

c)

Mean of 3 pigs due to mortality

Table 13 shows percent recovery of <u>T. hyodysenteriae</u> from rectal swabs at each sampling period. Hygromycin A treatment group A, B and C were observed the percent recovery of infected microorganisms at average 10%, 5% and 13%, respectively, which is almost same as of 8% in carbadox treatment group D, and is lower percent recovery of 36% in nonmedicated group E.

Table 11 show necropsy results.

In nonmedicated group E, all pigs were revealed lesions in large intestine, and in other groups, lesions were found 30% in group A, 70% in group B, 40% in group C and 10% in group D. As shown in Table 14, degree of lesions in groups A-C is slighter than that of in group E.

As illustrated hereinabove, hygromycin A was confirmed to have a curative effect on swine dysentery even at low doses of 1 g/ton feed as same level as of 50 g/ton feed of carbadox, and hence hygromycin A is more useful for cure and prevention of swine dysentery than that of carbadox.

**T a b l e   1 3**  Percent recovery of _Treponema_  _hyodysenteriae_ from rectal swabs

| Treatment [a] | Group No. | | Pigs positive for _Treponema_  _hyodysenteriae_ on postchallenge day(%) | | | | | Mean of postchallenge sampling days |
|---|---|---|---|---|---|---|---|---|
| | | | −5 | 7 | 14 | 21 | 28 | |
| A | 6 | | 0 | 0 | 0 | 20 | 0 | 5 |
| | 9 | | 0 | 20 | 20 | 0 | 20 | 15 |
| | | Mean | 0 | 10 | 10 | 10 | 10 | |
| B | 1 | | 0 | 20 | 0 | 0 | 20 | 10 |
| | 8 | | 0 | 0 | 0 | 0 | 0 | 0 |
| | | Mean | 0 | 10 | 0 | 0 | 10 | 5 |
| C | 2 | | 0 | 0 | 0 | 80 | 0 | 20 |
| | 5 | Mean | 0 | 0 | 20 | 0 | 0 | 5 |
| | | | 0 | 0 | 10 | 40 | 0 | 13 |
| D | 4 | | 0 | 20 | 20 | 0 | 0 | 10 |
| | 7 | | 0 | 0 | 0 | 25 | 0 | 6 |
| | | Mean | 0 | 10 | 10 | 11 | 0 | 8 |
| E | 3 | | 0 | 40 | 50 | 25 | 33 | 38 |
| | 10 | | 0 | 20 | 50 | 25 | 25 | 35 |
| | | Mean | 0 | 30 | 50 | 25 | 43 | 36 |

Table 13  ( continued )

a)

A  =  1 0 g／T  Hygromycin  A

B  =     5 g／T  Hygromycin  A

C  =     1 g／T  Hygromycin  A

D  =  5 0 g／T  Carbadox

E  =  Nonmedicated

0243717

Table 14 Necropsy Results

| Treatment[a] | Number of Pigs with the Following Lesions Observed in the Large Intestine | | | | | | | | | Lesion Factor[b] | Number of pigs without Lesions |
|---|---|---|---|---|---|---|---|---|---|---|---|
| | Congestion | Edema | Fibrinous Material | Bloody Mucosa | Watery contents | Mucous | Enlarged submucosal Glands | Thin Cecal or Colon Wall | Necrosis | | |
| A | 3 | 1 | 0 | 0 | 0 | 0 | 0 | 0 | 0 | 4 | 7 |
| B | 6 | 2 | 0 | 0 | 0 | 0 | 0 | 0 | 0 | 8 | 3 |
| C | 3 | 2 | 1 | 0 | 1 | 1 | 0 | 0 | 0 | 8 | 6 |
| D | 1 | 1 | 1 | 1 | 1 | 1 | 0 | 0 | 1 | 7 | 9 |
| E | 10 | 3 | 5 | 4 | 8 | 4 | 6 | 0 | 3 | 43 | 0 |

a)

1 0 pigs/treatment

b)

Lesion factor = value obtained by adding together the number of pigs with lesions present

for each parameter

c)

A = 1 0 g/T Hygromycin A

B = 5 g/T Hygromycin A

-37-

Table 14 ( continued )

C  =    1 g／T   Hygromycin  A

D  =    5 0 g／T   Carbadox

E  =    Nonmedicated

**Example 7**

The test is conducted using 40 crossbred pigs approximately 6 weeks of age, weighing 12.8 kg at receipt. Pigs are assigned to one of the treatments outlined in the Table below, stratified by body weight and sex, and randomly assigned to pens within a randomized block design. Sexes intermixed within pens and proportioned among treatment groups. Treatments groups are randomly assigned to pens within experimental blocks to alleviate location variables (see Table 9). Each treatment group consists of two replicate pens with five pigs per pen.

| Treatment Designation | Drug in Feed | Drug Level (g/T) | Induced Dysentery | No. Replicate Pens | No. Pigs Per Pen | Total No. Pigs Per Treatment |
|---|---|---|---|---|---|---|
| A | Hygromycin A | 20 | Yes | 2 | 5 | 10 |
| B | Hygromycin A | 10 | Yes | 2 | 5 | 10 |
| C | Carbadox | 50 | Yes | 2 | 5 | 10 |
| D | None | 0 | Yes | 2 | 5 | 10 |

All pigs are adjusted to the test facility for four days prior to their challenge with 100 ml of T. hyodysenteriae (T. hyo) broth culture. Respective treatment diets are provided 1 hr postchallenge and continued ad libitum for three weeks followed by a two week nonmedicated period. Clinical signs are noted and recorded for each pig each day postchallenge. All pigs are necropsied on day 35 postchallenge to assess gross lesions of the large intestine. Feed consumption and individual pig body weight measurements and rectal swabs for T. hyo isolation are taken prechallenge and weekly postchallenge.

Table 15. Number of "pig days" with general appearance score of 1, 2, 3 or 4[a] for each replicate of each treatment group

| Treatment[b] | Pen | No. Pig Days with General Appearance Score of: | | | |
|---|---|---|---|---|---|
| | | 1 | 2 | 3 | 4 |
| A | 1 | 174 | 1 | 0 | 0 |
| | 4 | 175 | 0 | 0 | 0 |
| | Total | 349 | 1 | 0 | 0 |
| B | 5 | 175 | 0 | 0 | 0 |
| | 8 | 174 | 1 | 0 | 0 |
| | Total | 349 | 1 | 0 | 0 |
| C | 2 | 175 | 0 | 0 | 0 |
| | 7 | 174 | 1 | 0 | 0 |
| | Total | 349 | 1 | 0 | 0 |
| D | 3 | 149 | 26 | 0 | 0 |
| | 6 | 125 | 50 | 0 | 0 |
| | Total | 274 | 76 | 0 | 0 |

[a] 1 = Normal
2 = Depressed
3 = Moribund
4 = Dead

[b] A = 20 g/ton hygromycin A T. hyo infected
B = 10 g/ton hygromycin A T. hyo infected
C = 50 g/ton Carbadox, T. hyo infected
D = nonmedicated, T. hyo infected

Pigs are challenged on day 5 and then provided respective treatment diets immediately postchallenge for a 21-day period followed by a 14-day nonmedication period.

Table 16. Number of "pig days" with hydration score of 1, 2, 3 or 4[a] for each replicate of each treatment group

| Treatment[b] | Pen | No. Pig Days with Hydration Score of: | | | |
|---|---|---|---|---|---|
| | | 1 | 2 | 3 | 4 |
| A | 1 | 175 | 0 | 0 | 0 |
| | 4 | 175 | 0 | 0 | 0 |
| | Total | 350 | 0 | 0 | 0 |
| B | 5 | 173 | 2 | 0 | 0 |
| | 8 | 173 | 2 | 0 | 0 |
| | Total | 346 | 4 | 0 | 0 |
| C | 2 | 174 | 1 | 0 | 0 |
| | 7 | 173 | 2 | 0 | 0 |
| | Total | 347 | 3 | 0 | 0 |
| D | 3 | 120 | 38 | 12 | 5 |
| | 6 | 117 | 27 | 19 | 12 |
| | Total | 237 | 65 | 31 | 17 |

[a]1 = Normal
2 = Slight gauntness of flanks and abdomen (mild)
3 = Gaunt, increased thirst, muscular weakness (moderate)
4 = Emaciated, very thirsty, very obvious muscular weakness (severe)

[b]A = 20 g/ton hygromycin A, T. hyo infected
B = 10 g/ton hygromycin A, T. hyo infected
C = 50 g/ton hygromycin A, T. hyo infected
D = nonmedicated, T. hyo infected

Pigs are challenged on day 5 and then provided respective treatment diets immediately postchallenge for a 21-day period followed by a 14-day nonmedicated period.

Table 17. Feed consumption (kg) per pen of pigs for each measurement period and overall

| Treat-menta | Pen | 4-day Adjust-ment | Postchallenge Days | | | | | 35 Day Total |
|---|---|---|---|---|---|---|---|---|
| | | | 0-7 | 8-14 | 15-21 | 22-28 | 29-35 | |
| A | 1 | 14.9 | 35.7 | 45.6 | 49.5 | 47.3 | 68.0 | 246.1 |
| | 4 | 15.9 | 37.4 | 46.5 | 47.3 | 48.5 | 65.3 | 245.0 |
| | Total | 30.8 | 73.1 | 92.1 | 96.8 | 95.8 | 133.3 | 491.1 |
| B | 5 | 13.9 | 39.5 | 46.4 | 51.0 | 51.0 | 61.9 | 249.8 |
| | 8 | 16.9 | 37.9 | 47.9 | 53.1 | 54.0 | 64.5 | 257.4 |
| | Total | 30.8 | 77.4 | 94.3 | 104.1 | 105.0 | 126.4 | 507.2 |
| C | 2 | 16.5 | 37.8 | 47.2 | 55.6 | 55.1 | 78.9 | 274.6 |
| | 7 | 18.6 | 39.8 | 49.7 | 51.6 | 54.1 | 58.0 | 253.2 |
| | Total | 35.1 | 77.6 | 96.9 | 107.2 | 109.2 | 136.9 | 527.8 |
| D | 3 | 15.5 | 37.2 | 30.5 | 40.7 | 43.7 | 53.6 | 205.7 |
| | 6 | 16.7 | 34.8 | 26.9 | 38.0 | 46.5 | 57.5 | 203.7 |
| | Total | 32.2 | 72.0 | 57.4 | 78.7 | 90.2 | 111.1 | 409.4 |

[a] A = 20 g/ton hygromycin A, T. hyo infected
B = 10 g/ton hygromycin A, T. hyo infected
C = 50 g/ton Carbadox, T. hyo infected
D = nonmedicated, T. hyo infected

Pigs are challenged on day 5 and then provided respective treatment diets immediately postchallenge for a 21-day period followed by a 14-day nonmedication period.

0243717

Table 18. Average per pig body weight (kg) at each measurement period

| Treatment[a] | Pen | Adjustment Day | | Postchallenge Day | | | | |
|---|---|---|---|---|---|---|---|---|
| | | 0 | 4 | 7 | 14 | 21 | 28 | 35 |
| A | 1 | 13.2 | 14.7 | 18.3 | 22.4 | 27.1 | 31.3 | 37.4 |
| | 4 | 12.3 | 14.3 | 17.8 | 22.8 | 26.5 | 31.4 | 37.2 |
| | Mean | 12.8 | 14.5 | 18.0 | 22.6 | 26.8 | 31.4 | 37.3 |
| | (S.D.) | (0.83) | (0.99) | (1.34) | (1.54) | (1.76) | (2.00) | (2.68) |
| B | 5 | 12.5 | 14.3 | 18.6 | 23.2 | 28.1 | 32.5 | 37.7 |
| | 8 | 13.1 | 14.9 | 19.2 | 23.9 | 28.4 | 33.0 | 38.2 |
| | Mean | 12.8 | 14.6 | 18.9 | 23.6 | 28.2 | 32.8 | 37.9 |
| | (S.D.) | (0.86) | (1.1) | (1.13) | (1.45) | (1.75) | (1.95) | (2.31) |
| C | 2 | 12.7 | 14.7 | 18.7 | 23.5 | 28.5 | 33.0 | 39.4 |
| | 7 | 12.9 | 14.9 | 19.1 | 24.3 | 28.8 | 33.6 | 38.9 |
| | Mean | 12.8 | 14.8 | 18.9 | 23.9 | 28.6 | 33.3 | 39.2 |
| | (S.D.) | (0.81) | (1.18) | (1.40) | (1.56) | (1.47) | (1.59) | (1.85) |
| D | 3 | 13.0 | 14.7 | 17.9 | 19.3 | 23.4 | 26.6 | 32.0 |
| | 6 | 12.5 | 14.4 | 17.3 | 18.2 | 22.1 | 25.9 | 31.6 |
| | Mean | 12.8 | 14.5 | 17.6 | 18.8 | 22.8 | 26.2 | 31.8 |
| | (S.D.) | (0.90) | (0.90) | (1.96) | (4.88) | (5.97) | (4.98) | (5.63) |

[a] A = 20 g/ton hygromycin A, T. hyo infected
B = 10 g/ton hygromycin A, T. hyo infected
C = 50 g/ton Carbadox, T. hyo infected
D = nonmedicated, T. hyo infected

Pigs are challenged on day 5 and then provided respective treatment diets immediately postchallenge for a 21-day period followed by a 14-day nonmedication period.

Table 19. Total body weight gain (kg) per pen of pigs for each measurement period and overall

| Treatment[a] | Pen | 4-Day Adjustment | Postchallenge Days | | | | | Overall Gain Postchallenge Days 0-35 |
|---|---|---|---|---|---|---|---|---|
| | | | 0-7 | 8-14 | 15-21 | 22-28 | 29-35 | |
| A | 1 | 7.2 | 18.2 | 20.4 | 23.3 | 21.4 | 30.5 | 113.8 |
| | 4 | 9.9 | 17.4 | 25.4 | 18.1 | 24.7 | 29.2 | 114.8 |
| | Total | 17.1 | 35.6 | 45.8 | 41.4 | 46.1 | 59.7 | 228.6 |
| | (Mean) | (8.6) | (17.8) | (22.9) | (20.7) | (23.1) | (29.9) | (114.3) |
| B | 5 | 8.8 | 21.6 | 23.2 | 24.4 | 22.2 | 25.7 | 117.1 |
| | 8 | 9.1 | 21.3 | 23.5 | 22.5 | 22.9 | 26.2 | 116.4 |
| | Total | 17.9 | 42.9 | 46.7 | 46.9 | 45.1 | 51.9 | 233.5 |
| | (Mean) | (9.0) | (21.5) | (23.4) | (23.5) | (22.6) | (26.0) | (116.8) |
| C | 2 | 10.1 | 19.6 | 24.4 | 24.8 | 22.7 | 31.9 | 123.4 |
| | 7 | 9.8 | 21.2 | 26.1 | 22.2 | 24.3 | 26.4 | 120.2 |
| | Total | 19.9 | 40.8 | 50.5 | 47.0 | 47.0 | 58.3 | 243.6 |
| | (Mean) | (10.0) | (20.4) | (25.3) | (23.5) | (23.5) | (29.2) | (121.8) |
| D | 3 | 8.2 | 15.9 | 7.4 | 20.5 | 15.6 | 27.2 | 86.6 |
| | 6 | 9.3 | 14.4 | 4.7 | 19.2 | 19.2 | 28.7 | 86.2 |
| | Total | 17.5 | 30.3 | 12.1 | 39.7 | 34.8 | 55.9 | 172.8 |
| | (Mean) | (8.8) | (15.2) | (6.1) | (19.9) | (17.4) | (28.0) | (86.4) |

[a] A = 20 g/ton hygromycin A, T. hyo infected
B = 10 g/ton hygromycin A, T. hyo infected
C = 50 g/ton hygromycin A, T. hyo infected
D = nonmedicated, T. hyo infected

Pigs are challenged on day 5 and then provided respective treatment diets immediately postchallenge for a 21-day period followed by a 14-day nonmedication period.

Table 20. Necropsy results of T. hyodysenteriae-challenged pigs at 35 days postchallenge[a]

Number of Pigs with the Following Lesions Observed in the Large Intestine

| Treatment[c] | Congestion | Edema | Lymph Node Swelling | Fibrinous Material | Bloody Mucosa | Watery Contents | Mucus | Enlarged Submucosal Glands | Thin Cecal or Colon Wall | Necrosis | Lesion Factor[b] | No. Pigs Without Lesions |
|---|---|---|---|---|---|---|---|---|---|---|---|---|
| A | 4 | 1 | 2 | 0 | 0 | 1 | 0 | 0 | 0 | 0 | 8 | 4 |
| B | 0 | 0 | 0 | 0 | 0 | 1 | 0 | 0 | 0 | 0 | 1 | 9 |
| C | 2 | 0 | 1 | 0 | 0 | 0 | 0 | 0 | 0 | 0 | 3 | 8 |
| D | 4 | 4 | 1 | 1 | 0 | 4 | 1 | 2 | 0 | 0 | 17 | 1 |

[a]10 pigs/treatment; all lesions were generally mild

[b]Value obtained by adding together the number of pigs with lesions present for each parameter

[c]A = 20 g/ton hygromycin, T. hyo infected
B = 10 g/ton hygromycin, T. hyo infected
C = 50 g/ton Carbadox, T. hyo infected
D = nonmedicated, T. hyo infected

Pigs are challenged on day 5 and then provided respective treatment diets immediately postchallenge for a 21-day period followed by a 14-day nonmedication period.

Table 21. Percent recovery of _T. hyodysenteriae_ from rectal swabs at each sampling period

| Treatment[a] | Pen No. | % of Pigs Positive for _T. hyodysenteriae_ on Postchallenge Day: | | | | | | Mean of 5 Sampling Days |
|---|---|---|---|---|---|---|---|---|
| | | -5 | 7 | 14 | 21 | 28 | 35 | |
| A | 1 | 0 | 0 | 0 | 20 | 20 | 0 | 8 |
| | 4 | 0 | 20 | 40 | 0 | 20 | 20 | 20 |
| | Mean | 0 | 10 | 20 | 10 | 20 | 10 | 14 |
| B | 5 | 0 | 0 | 20 | 0 | 100 | 0 | 24 |
| | 8 | 0 | 0 | 20 | 0 | 60 | 0 | 16 |
| | Mean | 0 | 0 | 20 | 0 | 80 | 0 | 20 |
| C | 2 | 0 | 60 | 40 | 80 | 20 | 0 | 40 |
| | 7 | 0 | 40 | 40 | 0 | 80 | 0 | 32 |
| | Mean | 0 | 50 | 40 | 40 | 50 | 0 | 36 |
| D | 3 | 0 | 20 | 60 | 60 | 40 | 0 | 36 |
| | 6 | 0 | 20 | 40 | 40 | 40 | 20 | 32 |
| | Mean | 0 | 20 | 50 | 50 | 50 | 10 | 34 |

[a] A = 20 g/ton hygromycin A, _T. hyo_ infected
B = 10 g/ton hygromycin A, _T. hyo_ infected
C = 50 g/ton Carbadox, _T. hyo_ infected
D = nonmedicated, _T. hyo_ infected

Pigs are challenged on day 5 and then provided respective treatment diets immediately postchallenge for a 21-day period followed by a 14-day nonmedication period.

WHAT IS CLAIMED IS:

1. A method of curing swine dysentery, comprising administering a therapeutically sufficient amount of hygromycin A to swine suffering from swine dysentery.

2. A method of preventing swine dysentery, comprising administering a prophylactically effective amount of hygromycin A to swine at risk of developing swine dysentery.

3. The method of Claim 1 or Claim 2 wherein the hygromycin A is administered orally.

4. The method of Claim 3 wherein the hygromycin A is administered orally as an additive in the drinking water.

5. The method of Claim 3 wherein the hygromycin A is administered orally as an additive in the feed.

6. A method of preventing and curing swine dysentery, comprising administering from about 0.02 mg to about 3.0 mg hygromycin A per kg of swine weight.

7. A composition for the control of swine dysentery, peripneumonia and swine plague in swine selected from the group consisting of drinking water containing from about 0.2 to about 30.0 mg hygromycin A per liter and swine feed containing from about 0.2 to about 20 mg hygromycin A per kg.

8. A composition for the prophylaxis and treatment of swine dysentery comprising from about 0.2 mg to about 30 mg per liter of hygromycin A dissolved in swine drinking water.

9. A composition for the prophylaxis and treatment of swine dysentery comprising from about 0.2 mg to about 20 mg per kg of hygromycin A in swine feed.

10. A method of controlling peripneumonia in swine comprising administering a pharmaceutically effective amount of hygromycin A to swine suffering from peripneumonia or at risk of developing peripneumonia.

11. A method of controlling swine plague in swine suffering from or at risk of developing swine plague comprising administering a pharmaceutically effective amount of hygromycin A to said swine.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 93, no. 13, 29th September 1980, page 152, column 2, abstract no. 143766k, Inst. Bioquim Macromal., Uni. auto. Madrid, SP; M. del C. GUERRERO et al.: "Hygromycin A, a novel inhibitor of ribosomal peptidyltransferase", & EUR. J. BIOCHEM 1980, 107(2), 409-414 | 1-11 | C 07 H   15/203 A 61 K   31/70 A 61 K   31/05 |
| | --- | | |
| A | KIRK-ORHMER ENCYCLOPEDIA OF CHEMICAL TECHNOLOGY, vol. 2, 3rd edition, John Wiley & Sons, New York, US; * page 846, lines 3-15, figure 120 * | 1-11 | |
| | --- | | **TECHNICAL FIELDS SEARCHED (Int. Cl.4)** |
| A | CHEMICAL ABSTRACTS, vol. 56, no. 6, 19th March 1962, abstract no. 6345, line f; "Antibiotics in feeds", & ANN. NUTRITION ALIMENT. 1961, 15, 75-89 * especially lines 8-143 * | 1-11 | C 07 H   15/00 A 61 K   31/00 |
| | --- | | |
| A | US-A-4 237 120  (AMERICAN CYANAMID CORP.) * claims 7, 29 * | 1-11 | |
| | ----- | | |

The present search report has been drawn up for all claims

| Place of search | Date of completion of the search | Examiner |
|---|---|---|
| BERLIN | 03-08-1987 | AVEDIKIAN P.F. |